# EUROPEAN PATENT APPLICATION

(11) **EP 2 912 995 A1**
(43) Date of publication of application: **02.09.2015**
(21) Application number: 14194640.0
(22) Date of filing: 08.09.2011
(51) Int. Cl.: A61B 1/002

(54) **Method and apparatus for illuminating a field of view of an optical system for generating three dimensional image information**

(62) Divisional of application: 11872152.1
(71) Applicant: FRONT STREET INVESTMENT MANAGEMENT INC. as manager for Front Street Diversified Income Class, Toronto ON M5E 1G4 (CA)
(72) Inventor: Mitchell, Thomas N., Bowen Island, British Columbia V0N 1G0 (CA); Shinkoda, Ichiro, Bowen Island, British Columbia V0N 1G1 (CA); Beckett, Martin, Bowen Island, British Columbia V0N 1G0 (CA)
(74) Representative: Hansson, Anders Max

(57) **Abstract**

An apparatus for generating three dimensional image information, the apparatus comprising: an optical system having an imaging path disposed to capture light within a field of view of the optical system; an image modulator operably configured to cause first and second images to be received through respective first and second imaging portions of the imaging path, said first portion of the imaging path having a first perspective viewpoint within the field of view, said second portion of the imaging path having a second perspective viewpoint within the field of view; an illuminator operably configured to alternate between: directing said illumination flux through said second portion of the imaging path to illuminate a first portion of the field of view of the optical system while said first image is being received; and directing said illumination flux through said first portion of the imaging path to illuminate a second portion of the field of view of the optical system, while said second image is being received; said first and second images together being operable to represent three dimensional spatial attributes of objects within the field of view.

## Description

### BACKGROUND OF THE INVENTION

### 1. Field of Invention

This invention relates generally to generating three-dimensional image information and more particularly to illuminating a field of view of an optical system for generating three-dimensional image information.

### 2. Description of Related Art

Optical instruments such as microscopes, endoscopes, and borescopes for viewing objects that are located in difficult to access locations will generally require illumination of the field of view of the instrument to facilitate formation of useable images.

In commonly owned United States Patent 6,151,164 entitled "Stereoscopic viewing system using a two dimensional lens system", a stereoscopic viewing system provides stereoscopic viewing using a two dimensional lens system, without the need for duplicated imaging paths and/or cameras. The stereoscopic views provide left and right perspective views that may be viewed or processed to provide a three-dimensional (3D) image representation. For an endoscope, light for illuminating the field of view may be coupled through optical fibers that are peripherally routed through a bore of the instrument.

### SUMMARY OF THE INVENTION

In accordance with one aspect of the invention there is provided a method for generating three dimensional image information through an optical system having an imaging path defined by a plurality of optical elements disposed to capture light within a field of view of the optical system. The method involves coupling the illumination flux through at least one illuminating portion of the imaging path to illuminate at least a portion of the field of view of the optical system, and receiving first and second images through respective first and second imaging portions of the imaging path, the first and second imaging portions being disposed adjacent to the at least one illuminating portion. The first imaging portion has a first perspective viewpoint within the field of view and the second imaging portion has a second perspective viewpoint within the field of view, the first and second images together being operable to represent three dimensional spatial attributes of objects within the illuminated field of view.

Coupling the illumination flux through the at least one illuminating portion of the imaging path may involve coupling the illumination flux through at least one generally rectangular portion of the imaging path defined between two generally parallel lines extending through the imaging path.

Coupling the illumination flux through the at least one generally rectangular portion of the imaging path may involve coupling the illumination flux through a generally rectangular portion surrounding an optical axis associated with the imaging path and receiving the first and second images may involve receiving first and second images through respective first and second imaging portions of the imaging path disposed on either side of the generally rectangular portion. Coupling the illumination flux through the at least one generally rectangular portion of the imaging path may involve coupling the illumination flux through first and second generally rectangular portions disposed on either side of an optical axis associated with the imaging path and receiving the first and second images may involve receiving first and second images through respective first and second imaging portions of the imaging path disposed between the first and second generally rectangular portions.

The plurality of optical elements have a generally circular cross-sectional shape and coupling the illumination flux through the at least one illuminating portion of the imaging path may involve coupling the illumination flux through first and second segments of the imaging path disposed on either side of an optical axis associated with the imaging path and receiving the first and second images may involve receiving first and second images through respective first and second imaging portions of the imaging path disposed between the first and second segments of the imaging path.

Coupling the illumination flux through the at least one illuminating portion of the imaging path may involve coupling the illumination flux through at least one generally circular portion of the imaging path.

Coupling the illumination flux through the at least one generally circular portion of the imaging path may involve coupling the illumination flux through a generally circular portion of the imaging path surrounding an optical axis of the imaging path and receiving the first and second images may involve receiving first and second images through respective first and second imaging portions of the imaging path disposed on either side of the generally circular portion.

Coupling the illumination flux through the at least one illuminating portion of the imaging path may involve coupling the illumination flux through at least one illuminating portion of a first optical element of the plurality of optical elements.

Coupling the illumination flux through the at least one illuminating portion of the first optical element may involve coupling the illumination flux through at least one illuminating portion of at least one of an objective lens, and a window disposed to transmit light from the field of view into the imaging path.

Coupling the illumination flux through the at least one illuminating portion may involve coupling the illumination flux through a centrally disposed opening in the at least one of the objective lens and the window.

Coupling the illumination flux through the at least one illuminating portion of the imaging path may involve receiving the illumination flux from an illumination source and guiding the illumination flux to the at least one illuminating portion of the imaging path.

Guiding the illumination flux may involve guiding the illumination flux through one of an optical fiber bundle and a waveguide.

The method may involve receiving the first and second images at an image sensor and causing the image sensor to generate first and second signals representing the respective first and second images.

The method may involve receiving the first and second signals at a display and causing the display to display the first and second images.

The method may involve selectively directing the first image to a left eye of an operator and selectively directing the second image to a right eye of the operator.

The display may be operably configured to simultaneously display each of the first and second images and may further involve preventing the first image from reaching the right eye of the operator and preventing the second image from reaching the left eye of the operator while permitting the first image to reach the left eye of the operator and permitting the second image to reach the right eye of the operator.

Receiving the first and second images through first and second imaging portions of the imaging path may involve alternating between preventing light from reaching the first imaging portion of the imaging path and preventing light from reaching the second imaging portion of the imaging path.

Receiving the first and second images may involve simultaneously receiving the first and second images through the first and second imaging portions of the imaging path and may further involve alternating between preventing light passing through the first imaging portion of the imaging path from being transmitted through the optical system, and preventing light passing through the second imaging portion the imaging path from being transmitted through the optical system.

Receiving the first and second images may involve simultaneously receiving the first and second images through the first and second imaging portions of the imaging path and simultaneously transmitting the first and second images through the optical system, simultaneously receiving the first and second images transmitted through the optical system at an image modulator, and causing the image modulator to selectively transmit the respective first and second images.

Causing the image modulator to selectively transmit the respective first and second images may involve causing the image modulator to alternately block light associated with the first image while transmitting light associated with the second image, and block light associated with the second image while transmitting light associated with the first image.

Causing the image modulator to selectively transmit the respective first and second images may involve causing the image modulator to modify a state of light associated with the first image to have a first optical state, modify a state of light associated with the second image to have a second optical state, and the first and second optical states may be operable to facilitate separation of the first and second images into first and second images at an image sensor disposed to receive the first and second images.

Causing the image modulator to modify the state of light associated with the first and second images to have respective first and second optical states may involve causing the image modulator to modify a polarization state of the respective first and second images.

In accordance with another aspect of the invention there is provided an apparatus for generating three dimensional image information. The apparatus includes an optical system having an imaging path disposed to capture light within a field of view of the optical system. The imaging path includes at least one illuminating portion operably configured to couple the illumination flux to illuminate at least a portion of the field of view of the optical system. The apparatus also includes an image modulator operably configured to cause first and second images to be received through respective first and second imaging portions of the imaging path, the first and second imaging portions being disposed adjacent to the at least one illuminating portion. The first imaging portion has a first perspective viewpoint within the field of view and the second imaging portion has a second perspective viewpoint within the field of view, the first and second images together being operable to represent three dimensional spatial attributes of objects within the illuminated field of view.

The at least one illuminating portion may include at least one generally rectangular portion of the imaging path defined between two generally parallel lines extending through the imaging path.

The at least one generally rectangular portion of the imaging path may include a generally rectangular portion surrounding an optical axis associated with the imaging path and the image modulator may be operably configured to cause the first and second images to be received through respective first and second imaging portions of the imaging path disposed on either side of the generally rectangular portion.

The at least one generally rectangular portion may include first and second generally rectangular portions disposed on either side of an optical axis associated with the imaging path and the image modulator may be operably configured to cause the first and second images to be received through respective first and second imaging portions of the imaging path disposed between the first and second generally rectangular portions.

The plurality of optical elements have a generally circular cross-sectional shape and the at least one illuminating portion of the imaging path may include first and second segments of the imaging path disposed on either side of an optical axis associated with the imaging path and the image modulator may be operably configured to cause the first and second images to be received through respective first and second imaging portions of the imaging path disposed between the first and second segments of the imaging path.

The at least one illuminating portion of the imaging path may include at least one generally circular portion of the imaging path.

The at least one generally circular portion of the imaging path may include a generally circular portion of the imaging path surrounding an optical axis of the imaging path.

The at least one illuminating portion of the imaging path may include at least one illuminating portion of a first optical element of the plurality of optical elements.

The at least one illuminating portion of the first optical element may include at least one illuminating portion of at least one of an objective lens, and a window disposed to transmit light into the imaging path.

The at least one illuminating portion of the objective lens may include a centrally disposed opening in the at least one of the objective lens, and the window.

The apparatus may include an illumination source, and an illumination path operably configured to receive the illumination flux from the illumination source and to guide the illumination flux to the illuminating portion of the imaging path. The illumination path may include one of an optical fiber bundle and a waveguide.

The apparatus may include an image sensor for receiving the first and second images and generating first and second signals representing the respective first and second images.

The apparatus may include a display for receiving the first and second signals and displaying the first and second images.

The apparatus may include an optical processor operably configured to selectively direct the first image to a left eye of an operator and to selectively direct the second image to a right eye of the operator.

The display may be operably configured to simultaneously display each of the first and second images and the optical processor may be operably configured to prevent the first image from reaching the right eye of the operator and to prevent the second image from reaching the left eye of the operator while permitting the first image to reach the left eye of the operator and permitting the second image to reach the right eye of the operator.

The image modulator may be disposed to alternate between preventing light from reaching the first imaging portion of the imaging path and preventing light from reaching the second imaging portion of the imaging path.

The optical system may be operably configured to simultaneously receive the first and second images through the first and second imaging portions of the imaging path and the image modulator may be operably configured to alternate between preventing light passing through the first imaging portion of the imaging path from being transmitted through the optical system, and preventing light passing through the second imaging portion the imaging path from being transmitted through the optical system.

The optical system may be operably configured to simultaneously receive the first and second images through the first and second imaging portions of the imaging path and to simultaneously transmit the first and second images through the optical system and the image modulator may be disposed to simultaneously receive the first and second images transmitted through the optical system and to selectively transmit the respective first and second images.

The image modulator may be operably configured to selectively transmit the respective first and second images by alternately blocking light associated with the first image while transmitting light associated with the second image, and blocking light associated with the second image while transmitting light associated with the first image.

The image modulator may be operably configured to selectively transmit the respective first and second images by modifying a state of light associated with the first image to have a first optical state, modifying a state of light associated with the second image to have a second optical state, and the first and second optical states may be operable to facilitate separation of the first and second images into first and second images at an image sensor disposed to receive the first and second images.

The image modulator may be operably configured to modify the state of light associated with the first and second images by modifying a polarization state of the respective first and second images.

In accordance with another aspect of the invention there is provided a method for generating three dimensional image information using an optical system having an imaging path disposed to capture light within a field of view of the optical system. The method involves alternating between receiving a first image through a first portion of the imaging path while directing the illumination flux through a second portion of the imaging path to illuminate a first portion of the field of view of the optical system. The first portion of the imaging path has a first perspective viewpoint within the field of view. The method also involves receiving a second image through a second portion of the imaging path while directing the illumination flux through the first portion of the imaging path to illuminate a second portion of the field of view of the optical system. The second portion of the imaging path has a second perspective viewpoint within the field of view. The first and second images together are operable to represent three dimensional spatial attributes of objects within the field of view.

Receiving the first and second images may involve receiving first and second images through an elongated imaging path operable to capture light from an inaccessible location.

The inaccessible location may be a location within a living body.

The method may involve generating an illumination flux and directing the illumination flux through the first and second portions of the imaging path to illuminate the respective first and second portions of the field of view may involve selectively actuating a modulator disposed in the imaging path to alternate between transmitting the first image while reflecting at least a portion of the illumination flux through the second portion of the imaging path to illuminate the first portion of the field of view, and transmitting the second image while reflecting at least a portion of the illumination flux through the first portion of the imaging path to illuminate the second portion of the field of view.

Selectively actuating the modulator may involve selectively actuating a mechanical shutter to move between a first position the shutter blocks transmission of the second image through the imaging path while reflecting the illumination flux through the imaging path to illuminate the second portion of the field of view, and a second position the shutter blocks transmission of the first image through the imaging path while reflecting the illumination flux through the imaging path to illuminate the first portion of the field of view.

Selectively actuating the modulator may involve selectively actuating a light valve to alternately block transmission of the second image through the imaging path while reflecting the illumination flux through the imaging path to illuminate the second portion of the field of view, and block transmission of the first image through the imaging path while reflecting the illumination flux through the imaging path to illuminate the first portion of the field of view.

Directing the illumination flux through the first and second portions of the imaging path to illuminate the respective first and second portions of the field of view may involve alternating between selectively actuating a first illuminator disposed to illuminate the first portion of the field of view while selectively actuating a modulator to transmit the first image through the imaging path, and selectively actuating a second illuminator disposed to illuminate the second portion of the field of view while selectively actuating the modulator to transmit the second image through the imaging path.

Selectively actuating the first and second illuminators may involve selectively actuating first and second illuminators disposed proximate an aperture plane of the optical system.

Selectively actuating the first and second illuminators may involve selectively actuating respective first and second light emitting diodes.

Selectively actuating the modulator may involve causing a shutter to move between first and second positions within the imaging path.

The first portion of the field of view of the optical system and the second portion of the field of view of the optical system have an overlapping region within the field of view.

Directing the illumination flux through the respective first and second portions of the imaging path may involve receiving the illumination flux from an illumination source and selectively guiding the illumination flux to the respective first and second portions of the imaging path.

Selectively guiding the illumination flux may involve guiding the illumination flux into an optical fiber bundle having first and second respective pluralities of fibers, the first plurality of fibers being disposed to illuminate the first portion of the field of view and the second plurality of fibers being disposed to illuminate the second portion of the field of view.

The method may involve selectively directing the first image to a left eye of the operator and selectively directing the second image to a right eye of the operator.

Selectively directing may involve presenting each of the first and second images to each of the left and right eyes of the operator, and preventing the first image from reaching the right eye of the operator and preventing the second image from reaching the left eye of the operator while permitting the first image to reach the left eye of the operator and the second image to reach the right eye of the operator.

Selectively directing may involve alternating between permitting the first image to be transmitted through the optical system and permitting the second image to be transmitted through the optical system while alternating between selectively permitting the first image to reach the left eye of the operator and selectively permitting the second image to reach the right eye of the operator.

In accordance with another aspect of the invention there is provided an apparatus for generating three dimensional image information. The apparatus includes an optical system having an imaging path disposed to capture light within a field of view of the optical system, an image modulator operably configured to cause first and second images to be received through respective first and second imaging portions of the imaging path, the first portion of the imaging path having a first perspective viewpoint within the field of view, the second portion of the imaging path having a second perspective viewpoint within the field of view, an illuminator operably configured to alternate between directing the illumination flux through a second portion of the imaging path to illuminate a first portion of the field of view of the optical system while the first image is being received, and directing the illumination flux through the first portion of the imaging path to illuminate a second portion of the field of view of the optical system, while the second image is being received, the first and second images together being operable to represent three dimensional spatial attributes of objects within the field of view.

The imaging path may include an elongated imaging path operable to capture light from an inaccessible location.

The inaccessible location may be a location within a living body.

The illuminator may include an illumination source operable to generate an illumination flux and the modulator may be operably configured to alternate between transmitting the first image while reflecting at least a portion of the illumination flux through the second portion of the imaging path to illuminate the first portion of the field of view, and transmitting the second image while reflecting at least a portion of the illumination flux through the first portion of the imaging path to illuminate the second portion of the field of view.

The modulator may include a mechanical shutter operably configured to move between a first position the shutter blocks transmission of the second image through the imaging path while reflecting the illumination flux through the imaging path to illuminate the second portion of the field of view, and a second position the shutter blocks transmission of the first image through the imaging path while reflecting the illumination flux through the imaging path to illuminate the first portion of the field of view.

The modulator may include a light valve operably configured to alternately block transmission of the second image through the imaging path while reflecting the illumination flux through the imaging path to illuminate the second portion of the field of view, and block transmission of the first image through the imaging path while reflecting the illumination flux through the imaging path to illuminate the first portion of the field of view.

The illuminator may include a first illuminator disposed to illuminate the first portion of the field of view while receiving the first image through the imaging path, and a second illuminator disposed to illuminate the second portion of the field of view while receiving the second image through the imaging path.

The first and second illuminators may be disposed proximate an aperture plane of the optical system.

The first and second illuminators may include respective first and second light emitting diodes.

The modulator may include a shutter operably configured to move between first and second positions within the imaging path.

The first portion of the field of view of the optical system and the second portion of the field of view of the optical system have an overlapping region within the field of view.

The illuminator may include an illumination source for generating the illumination flux, and a guide operable to selectively guide the illumination flux to the respective first and second portions of the imaging path.

The guide may include an optical fiber bundle having first and second respective pluralities of fibers, the first plurality of fibers being disposed to illuminate the first portion of the field of view and the second plurality of fibers being disposed to illuminate the second portion of the field of view.

The apparatus may include provisions for selectively directing the first image to a left eye of the operator and provisions for selectively directing the second image to a right eye of the operator.

The provisions for selectively directing may include provisions for presenting each of the first and second images to each of the left and right eyes of the operator, and provisions for preventing the first image from reaching the right eye of the operator and preventing the second image from reaching the left eye of the operator while permitting the first image to reach the left eye of the operator and the second image to reach the right eye of the operator.

The modulator is operably configured to alternate between permitting the first image to be transmitted through the optical system and permitting the second image to be transmitted through the optical system and the provisions for selectively directing may include provisions for alternating between selectively permitting the first image to reach the left eye of the operator and selectively permitting the second image to reach the right eye of the operator.

Other aspects and features of the present invention will become apparent to those ordinarily skilled in the art upon review of the following description of specific embodiments of the invention in conjunction with the accompanying figures.

### BRIEF DESCRIPTION OF THE DRAWINGS

In drawings which illustrate embodiments of the invention,
- Figure **1**: is a side view of an apparatus for generating three dimensional image information according to a first embodiment of the invention;
- Figure **2**A **- 2**D: are a series of cross sectional views of the apparatus shown in Figure **1** taken along the line **2 - 2,** in accordance with various embodiments of the invention;
- Figure **3**: is an enlarged side view of two portions of the apparatus shown in Figure **1****;**
- Figure **4**: is a perspective view of an image modulator and image sensor used in the apparatus shown in Figure **1****;**
- Figure **5**: is a partially cut-away perspective view of a liquid crystal device used in the modulator shown in Figure **4****;**
- Figure **6**: is a schematic view of an optical system of the apparatus shown in Figure **1** taken along the line **6-6** shown in Figure **2**A;
- Figure **7**: is a side view of an apparatus for generating three dimensional image information according to an alternative embodiment of the invention;
- Figure **8**: is block diagram of a spatial modulator used in the apparatus shown in Figure **7****;**
- Figure **9**: is a graphical depiction of control signals for controlling the spatial modulator shown in Figure **8****;** and
- Figure **10**: is a side view of an apparatus for generating three dimensional image information according to another alternative embodiment of the invention.

### DETAILED DESCRIPTION

In some applications, it may be desirable to reduce the diameter of an optical instrument or increase the **3**D depth perception available from the stereoscopic images for a given diameter of the optical instrument. Illuminating the field of view represents a significant challenge in such applications.

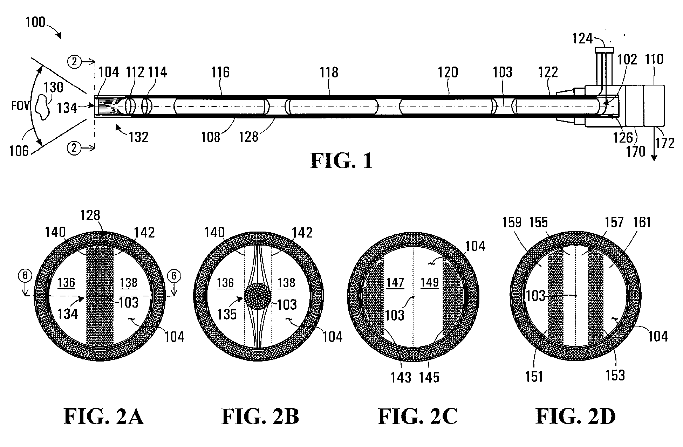

Referring to Figure **1****,** an apparatus for generating three dimensional image information according to a first embodiment of the invention is shown generally at **100.** The apparatus **100** includes an optical system **102** having an imaging path defined by a plurality of optical elements. The plurality of optical elements defining the imaging path are disposed within an elongated housing **108** and include a first optical element **104,** which in this embodiment is an objective lens, field lenses **112** and **114,** and a plurality of relay lenses **116 - 122.** In other embodiments the first optical element **104** may be a window and the lens **112** may be configured to act as an objective lens. Other elements in the plurality of optical elements may vary from that shown in Figure **1****.**

The apparatus **100** also includes an image sensor **110,** which in this embodiment is located some distance away from the objective lens **104.** The objective lens **104** is disposed to capture light within a field of view (FOV) **106** of the optical system and to transmit the light along the imaging path through the field lenses **112** and **114,** and through the plurality of relay lenses **116 - 122** to the image sensor **110.**

The apparatus **100** also includes an illumination coupler **124** for receiving an illumination flux from an external illumination source (not shown). The apparatus **100** also includes an illumination conduit **126,** which is disposed within the housing **108** and extends along the length thereof. In the embodiment shown, the illumination flux is coupled through the illumination conduit **126** using an optical fiber bundle **128.**

In the embodiment shown in Figure **1****,** the apparatus **100** and optical system **102** are configured as an endoscope and the housing **108** is dimensioned and configured for insertion inside a living body to enable distally located objects **130** to be viewed. The relay lenses **116 - 122** in the embodiment shown are configured as Hopkins type cemented doublet rod lenses, but other configurations of field lenses and/or relay lenses may be used to transmit the image from the distally located objective lens **104** to the image sensor **110.**

Alternatively, in other embodiments, optical system **102** may be configured to implement an alternative imaging system such as a microscope, borescope, or other optical system for generating 3D image information.

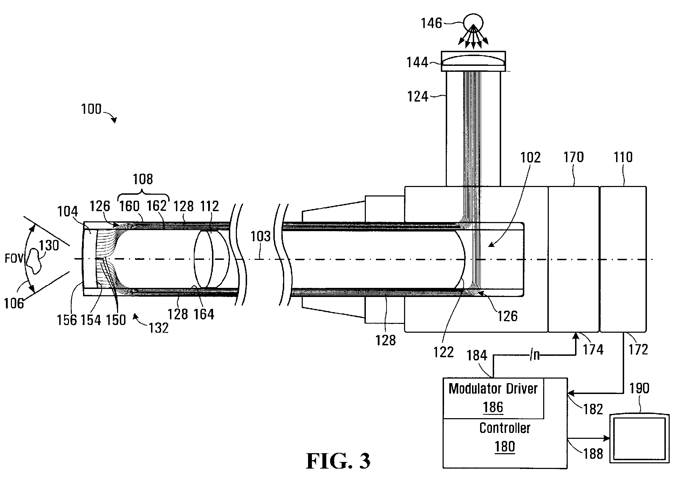

The optical system **102** is shown in cross sectional-view in Figures **2**A - **2**D and a distal portion **132** of the optical system **102** including the objective lens **104** is shown in enlarged plan view in Figure **3****.** Referring to Figure **2**A the imaging path includes an illuminating portion **134** defined between two generally parallel lines **140** and **142** extending through the imaging path. In this embodiment, the illuminating portion **134** has a generally rectangular shape and surrounds an optical axis **103** associated with the imaging path.

Referring to Figure **3****,** the illumination coupler **124** includes a collimating lens **144,** which is configured to receive the illumination flux from the external illumination source **146** and direct the flux to the fiber bundle **128.** The fiber bundle **128** extends through the illumination conduit **126** and terminates in a plurality of individual fibers **150** proximate the objective lens **104.** Each fiber **150** in the bundle **128** couples a portion of the illumination flux from the source through the illuminating portion of the objective lens **104.** The objective lens **104** couples the illumination flux through the illuminating portion to illuminate at least a portion of the field of view **106** of the optical system **102.** While the illumination embodiment shown in Figure **2**A has been shown implemented in Figure **3****,** any of the embodiments shown in Figure **2**B to Figure **2**D may equally well be implemented.

Referring back to Figure **2**A, the imaging path includes a first imaging portion **136** and a second imaging portion **138,** which are disposed on either side of the generally rectangular illuminating portion **134.** The first imaging portion **136** has a first perspective viewpoint within the field of view **106** and the second imaging portion **138** has a second perspective viewpoint within the field of view.

Referring to Figure **2**B, in another embodiment the illuminating portion of the objective lens **104** may include a generally circular portion **135** surrounding an optical axis **103** of the imaging path. The circular portion **135** illuminates the field of view and the first and second images are received through respective first and second imaging portions **136** and **138** of the imaging path disposed on either side of the generally circular portion. In the embodiment shown in Figure **2**A the generally rectangular illumination portion **134** increases the available illuminating area over the generally circular portion **135** shown in Figure **2**B and thus accommodates a greater number of and/or greater diameter of fibers, thus potentially increasing an illumination intensity provided by the illumination flux that is coupled through the illuminating portion **134.**

Referring to Figure **2**C, in another embodiment the illuminating portion includes a first segment **143** and a second segment **145** disposed on either side of the optical axis **103** of the imaging path, and the imaging path includes respective first and second imaging portions **147** and **149** disposed between the first and second segments.

Referring to Figure **2**D, in yet another embodiment the illuminating portion includes a first generally rectangular portion **151,** and a second generally rectangular portion **153** disposed on either side of the optical axis **103,** and the imaging path includes respective first and second imaging portions **155** and **157** of the imaging path disposed between the first and second generally rectangular portions. Further portions **159** and **161** of the imaging path may also be used to generate **3**D information.

In other embodiments the illuminating portion **134** may be otherwise configured to illuminate the field of view, while a remaining portion of the imaging path includes respective first and second imaging portions for the generating first and second images.

Referring to Figure **3****,** the objective lens **104** includes a first surface **154** and a second surface **156,** and in this embodiment the fibers **150** of the fiber bundle **128** terminate at the first surface **154,** and illumination guided through the fiber bundle passes through the objective lens for illuminating the field of view **106.** In other embodiments, the objective lens **104** may include an opening (not shown) for receiving the fiber bundle and the fiber bundle **128** may terminate at the second surface **156.** Such an opening may be formed through the objective lens **104,** in which case an additional window may be required to cover terminal ends of the fiber bundle **128.** In one embodiment, the objective lens **104** may include two separate lens segments, which are spaced apart to form the opening. Alternatively, the opening may only be formed partway through the objective lens **104** and the illumination flux may pass through a remaining portion of the objective lens to reach second surface **156.**

In the embodiment shown, the illumination conduit **126** is defined between an outer sheath **160** and an inner tube **162,** which together make up the housing **108.** The inner tube 162 includes an inside bore **164,** which is dimensioned to receive the lenses **104,** and **112 - 122** such that the respective lenses are centered within the bore in accordance with necessary optical tolerances. In one embodiment the inner tube **162** has a diameter of about **6** mm and the outer sheath **160** has a diameter of about **10** mm. Spacing between the lenses **104,** and **112** - **122** may be provided by tubular spacers (not shown) that are also accommodated within the bore **164** and extend between adjacent lenses. The illumination conduit **126** provides an annular cylindrical volume surrounding the inner tube **162** for routing the fibers **150** of the fiber bundle **128.**

Alternatively, in other embodiments the surfaces of the outer sheath **160** and inner tube **162** that define the illumination conduit **126** may be coated to cause the conduit to conduct an illumination flux along the conduit toward the objective **104,** thus acting as an illumination waveguide.

The apparatus **100** also includes an image modulator **170** disposed between the optical system **102** and the image sensor **110.** The image modulator **170** includes an input **174** for receiving a modulator control signal. The image modulator **170** is operably configured to cause the first and second images to be received through the respective first and second imaging portions **136** and **138** (shown in Figure **2**A) of the objective lens **104,** in response to the modulator control signal. The first and second images received through the optical system **102** are selectively directed through the modulator **170** to impinge on the image sensor **110.** As noted above, the first imaging portion **136** has a first perspective viewpoint within the field of view **106** and the second imaging portion **138** has a second perspective viewpoint within the field of view and the first and second images together include information that facilitates representation of three dimensional spatial attributes the object **130** within the illuminated field of view **106.**

The apparatus **100** further includes a controller **180** having an input **182** in communication with the output **172** of the image sensor **110** for receiving the image signal. The controller **180** also includes an output **184** in communication with the modulator input **174** for producing the modulator control signal and an output **188** for generating signals for driving a display **190.** In one embodiment the controller **180** may be implemented using a processor circuit such as a microprocessor or micro-controller, for example.

Since the first imaging portion **136** of the objective lens **104** is offset with respect to the optical axis **103,** the first image has a perspective viewpoint from one side of the object **130.** Similarly, the second image has a perspective viewpoint from the other side of the object **130.** When the first and second images are selectively directed to respective right and left eyes of a user, the user will be able to discern **3**D information from the images, in much the same way that the user would be able to discern **3**D information when viewing the actual object.

In one embodiment, the first and second images may be alternately displayed as separate video fields on the display **190.** Various types of active and passive eyewear are available for directing such displayed first and second images and to the user's eyes. Passive types of eyewear generally rely on additional wavelength or polarization processing of the images to enable passive filter elements in the eyewear to separate the images. Active types of eyewear generally include a receiver for receiving a synchronization signal from a display to alternately permit transmission of the first and second images to the respective left and right eyes. Alternatively, the first and second images may be processed to match up identifiable features in the respective images and to determine lateral shifts between the identified features. The determined lateral shifts along with a knowledge of the imaging parameters of the optical system **102** may be used to calculate a difference in depth between points on an object or between objects at different depths.

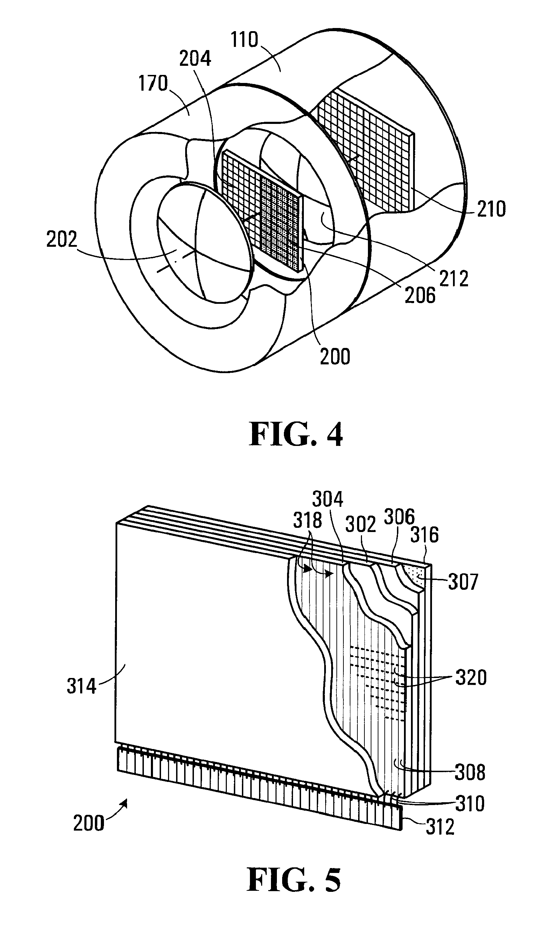

The image modulator **170** and image sensor **110** are shown in greater detail in Figure **4****.** Referring to Figure **4****,** in this embodiment the image modulator **170** includes a liquid crystal device (LCD) **200** and a lens **202** disposed to receive the first and second images from the optical system **102** and to direct the images onto the LCD. In general the lens **202** may comprise a plurality of lens elements, and in one embodiment the lens **202** may be selected to define an aperture plane for the optical system **102** at or proximate to the LCD **200.** Advantageously, locating the LCD **200** at an aperture plane reduces image formation problems such as vignetting of the respective first and second images.

In the embodiment shown the image sensor **110** comprises a charge coupled device array (CCD) **210,** which is coupled to the output **172** (shown in Figure **1** and Figure **3**) of the image sensor for generating an image signal representing the first and second images. The image sensor **110** also includes a lens **212** for forming the first and second images on the CCD **210.** In one embodiment the lens **212** may include a plurality of lenses operably configured to format the first and second images in to fill an active area of the CCD **210.**

The LCD **200** is responsive to the modulator control signal received at the input **174** (shown in Figure **3**) to alternately actuate first and second regions **204** and **206** of the LCD **200** to transmit light to form the respective first and second images. The LCD **200** is shown in greater detail in Figure **5****.** Referring to Figure **5****,** the LCD **200** includes a liquid crystal material layer **302** disposed between a first glass plate **304** and a second glass plate **306.** The first glass plate **304** includes a plurality of transparent electrodes **308** formed on a surface thereof and arranged in columns. Each electrode **308** has an associated connector **310,** which may be a wire-bonded or flexible circuit connection, for example. The connectors **310** connect to a header **312,** which in turn facilitates connection to the output **184** of the controller **180** (shown in Figure **3**). The second glass plate **306** includes a transparent area electrode **307,** which extends across a surface of the second glass plate and acts as a common electrode. Each transparent electrode **308** defines an element between the electrode and the transparent area electrode **307,** thus defining a plurality of elements **318** that may be separately actuated by providing a drive potential between the corresponding electrode **308** and the area electrode **307.**

The LCD **200** also includes a first polarizer **314,** having a first linear polarization property (in this case vertical polarization). The first polarizer **314** overlays the plurality of transparent electrodes **308.** The LCD **200** further includes a second polarizer **316** overlaying the second electrode **307** and having a second linear polarization property (in this case horizontal polarization). In Figure **5** the various layers are not necessarily shown to scale.

Referring back to Figure **3****,** the controller **180** includes a modulator driver **186** for generating the drive signals at the output **184.** In the embodiment shown, the modulator driver is configured to drive the LCD modulator **200** shown in Figure **5****,** and the output **184** thus has "n" output channels corresponding to the number of elements **318** of the image modulator **170.** The output **184** thus provides an individual drive signal for each electrode **308** of the LCD **200.** The drive signals are coupled to the respective electrodes **308** via the header **312** and connectors **310,** with the common electrode **307** acting as a ground connection. In one embodiment the drive voltage may be a **50**% duty cycle square wave varying between a voltage *V*⁺ and *V*⁻, where the voltages are selected within a range of safe operating voltages to provide sufficient contrast between transmission and blocking of light impinging on the LCD **200.** In the embodiment shown in Figure **5****,** the plurality of columnar elements **318** facilitate changes to the extent of the regions **204** and **206** shown in Figure **4****.** However in other embodiments, the LCD **200** may be configured to include only two columnar elements, each element having an extent of approximately **50**% of the front surface of the LCD **200.**

In operation, the first polarizer **314** transmits light having a vertical polarization. In this embodiment the liquid crystal material **302** is selected so that in its relaxed phase (un-actuated) the polarization of light passing through the crystal is unaffected and the second polarizer **316** thus blocks the light. When actuated by the drive voltage applied to any of the electrodes **308,** a portion of the liquid crystal material layer **302** underlying the electrode causes light transmitted through the layer to undergo a **90°** change in polarization, thus passing through the second polarizer **316** and the LCD **200.** By alternately generating drive signals for first and second pluralities of the electrodes **308,** the LCD **200** thus alternates between blocking and transmitting light at the first and second regions **204** and **206** respectively.

In an alternative embodiment the polarizers **314** and **316** may both be vertically polarized, such that the LCD Modulator is transmissive when no actuation voltage is applied between the electrodes **308** and **307.** In this case, when an element is actuated, the liquid crystal material causes the light to undergo a **90°** change in polarization thus causing elements **318** to block transmission of light.

In another embodiment, the electrodes **308** of the LCD **200** may be sub-divided into a plurality of pixels (shown in broken line at **320**) and connector **310** and header **312** may be configured to individually drive each pixel **320.** In operation, column of elements **318** may be actuated by actuating each of the pixels **320** in the column. Alternatively, it may be desired to actuate pixels in other than columnar patterns. For example, the liquid crystal device (LCD) **200** may be disposed at an aperture plane of the of the optical system **102** and the pixels **320** may be actuated to cause aperturing of the image at the aperture plane by actuating pixels in a circular or semi-circular pattern.

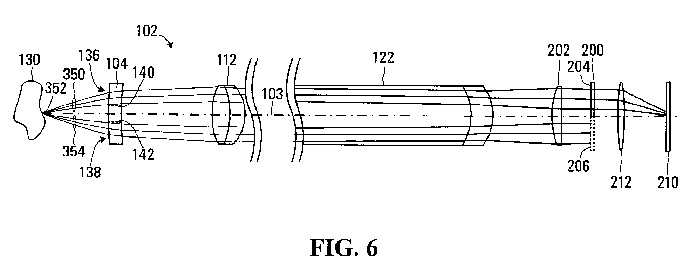

The operation of the apparatus **100** in generating **3**D image information is described further with reference to Figure **6****.** Referring to Figure **6****,** the optical system **102** of the apparatus **100** is shown in top view and portions of the housing **108,** illumination coupler **124,** image modulator **170,** and image sensor **110** are omitted for sake of clarity. The fiber bundle **128** (shown in Figure **3**) provides illumination of the field of view as described above. A first light ray bundle **350** emanating from a point **352** on the object **130** is captured through the first imaging portion **136** of the optical system **102** and after being relayed through the optical system by the lenses **112 - 122** is directed onto the first region **204** of the LCD **200** by the lens **202.** Similarly, a second light ray bundle **354** emanating from the point **352** on the object **130** is captured by the second imaging portion **138** of the optical system **102** and after being relayed through the optical system by the lenses **112 - 122** is directed onto the second region **206** of the LCD **200** by the lens **202.**

In the state shown in Figure **6****,** the first region **204** of the LCD **200** is actuated to permit light from the first imaging portion **136** of the objective lens **104** to be transmitted through the lens **212** to the CCD **210** for forming the first image. At the same time the second region **206** of the LCD **200** is actuated to prevent light from the second imaging portion **138** of the objective lens **104** from being transmitted. When the LCD **200** is actuated to cause the first region **204** to prevent light transmission and to cause the second region **206** to permit light transmission, light captured through the second imaging portion **138** of the objective lens **104** is transmitted through the second region **206** of the LCD **200** and a second image is formed on the CCD **210.**

In this embodiment, the respective first and second regions **204** and **206** have approximately the same extent (i.e. **50**% of the extent of the LCD **200**)**.** In other embodiments the first and second portions may be greater than or less than **50%** of the extent of the LCD **200.** In general, a level of **3**D depth discernment provided by the images is greater when the perspective viewpoints for each of the first and second images are spaced apart by a greater distance from the optical axis **103.**

Referring back to Figure **3****,** in the embodiment shown the illumination flux coupled through the centrally disposed illuminating portion **134** (shown in Figure **2**A) of the optical system **102** illuminates the field of view **106** for capturing the first and second images of the object **130.** In the case of endoscopy, the outside diameter of the housing **108** of the apparatus **100** is generally constrained by the need to provide access to difficult to reach portions of a patients body, and in such cases the diameter of the objective lens **104** is similarly constrained. Coupling the illumination flux through the centrally disposed illuminating portion **134** has the effect of spacing the first imaging portion **136** and second imaging portion **138** (shown in Figure **2**A) outwardly from the optical axis **103,** thereby providing a greater level of **3**D depth in the information in the first and second images. Use of the illuminating portion **134** for coupling illumination into the field of view of the apparatus **100,** as shown in Figure **2**A for example, also allows the objective lens **104** to occupy a larger diameter within the housing **108** than would be the case if the illumination flux were to be coupled through a peripheral portion of the housing **108,** as in conventional endoscopes. This allows a correspondingly larger diameter of the objective lens **104,** which increases the area of the first and second imaging portions **136** and **138** facilitating capture of more light. The illumination embodiments shown in Figures **2**B to **2**D would also provide similar benefits.

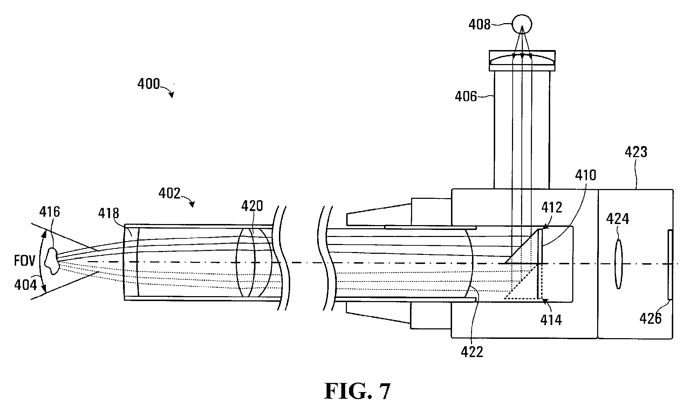

Referring to Figure **7****,** an apparatus for generating three dimensional image information according to a second embodiment of the invention is shown generally at **400.** The apparatus **400** includes an optical system **402** having an imaging path disposed to capture light within a field of view **404** of the optical system. The optical system **402** includes an objective lens **418,** and a plurality of other lenses of which a field lens **420** and a relay lens **422** are shown in Figure **7****.** The apparatus **400** also includes an illumination coupler **406** for receiving an illumination flux from an external illumination source **408.** The apparatus **400** further includes a spatial modulator **410.** In this embodiment the spatial modulator **410** is moveable between a first position **412** shown in solid lines and a second position **414** shown in broken outline.

When the spatial modulator **410** is in the first position **412,** the illumination flux is directed through a first portion of the imaging path to illuminate a first portion of the field of view **404.** When the spatial modulator **410** is in the second position **414,** the illumination flux is directed through a second portion of the imaging path to illuminate a second portion of the field of view **404.** The first and second portions of the field of view **404** are thus alternately illuminated through respective first and second portions of the imaging path".

The apparatus **400** also includes an image sensor **423** for receiving images produced by the optical system **402.** In this embodiment, the image sensor includes a lens **424** disposed to direct the image onto a CCD **426.**

When the spatial modulator **410** is in the second position **414,** the second portion of the field of view **404** is illuminated, and the first portion of the imaging path receives light captured from within the field of view **404.** The captured light is relayed through the optical system **402** by the lenses **(420 - 422)** and is received at the CCD **426** to form the first image. The first portion of the imaging path has a first perspective viewpoint within the field of view **404.** While the first portion of the field of view **404** is being illuminated, the second portion of the imaging path receives light captured from within the field of view **404.** The captured light is relayed through the optical system **402** by the relay lenses and is received at the CCD **426** to form the second image. The second portion of the imaging path has a second perspective viewpoint within the field of view **404.** The first and second images are together operable to represent three dimensional spatial attributes of the object **416.**

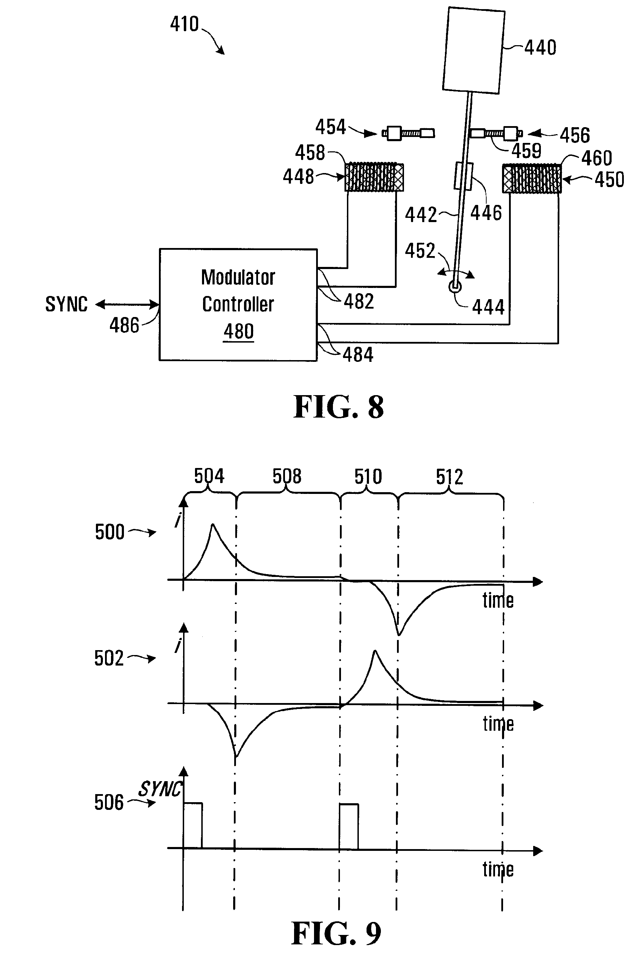

The spatial modulator **410** is shown in greater detail in Figure **8****.** Referring to Figure **8****,** the spatial modulator **410** includes a reflector **440** mounted on an arm **442.** The arm **442** is mounted on a pivot **444** to provide for side-to-side motion of the arm. The arm **442** also includes a magnet **446** mounted partway along the arm. The magnet **446** is disposed between first and second electromagnets **448** and **450.** The arm **442,** pivot **444,** and the electromagnets **448** and **450,** together make up a mechanical actuator operable to produce a force for moving the reflector **440** side-to-side in the direction of the arrow **452** between a pair of stops **454** and **456,** which respectively define first and second positions of the arm **442** and the reflector **440.** The stops **454** and **456** each comprise a threaded portion **459** that provides for adjustment for aligning the reflector **440** to direct the illumination flux along the first and second respective portions of the optical system **402.** In one embodiment the stops **454** and **456** may be coupled to an electrical actuator (not shown), thus facilitating alignment of the reflector **440** without requiring access to the modulator **410.**

For driving the spatial modulator **410,** the controller **180** of the embodiment shown in Figure **1** may be replaced by the modulator controller **480** shown in Figure **8****.** The modulator controller **480** includes a first pair of outputs **482** for driving a coil **458** of the first electromagnet **448** and a second pair of outputs **484** for driving a coil **460** of the second electromagnet **450.**

In operation, the modulator controller **480** generates a SYNCH signal internally, and in response to the SYNCH signal, generates current waveforms at the outputs **482** and **484** for driving the respective coils **458** and **460.** The currents through the respective coils **458** and **460** cause forces to be exerted on the arm **442** to move toward a desired stop **456** or **454.** Advantageously, the modulator controller **480** may be implemented as a push-pull controller where one of the electromagnets **448** and **450** provides an attractive force on the magnet **446,** while the other of the electromagnets provides a repulsion force.

Exemplary waveforms of a current drive provided to the coils **458** and **460** to cause the arm **442** to move toward the first electromagnet **448** are shown graphically in Figure **9****.** The current waveform through the coil **458** is shown at **500** and the current waveform through the coil **460** is shown at **502.** The SYNCH signal pulse waveform is shown at **506.** A rising edge of the SYNCH signal **506** defines a start time of a first time period **504,** in which the current **500** rises rapidly to produce an attractive force on the arm **442.** The attractive force overcomes the inertia of the arm **442** and causes the arm to accelerate away from the stop **456** and the second electromagnet **450.** During the first time period **504** the current **502** is initially at zero and once the arm **442** begins to accelerate, the current **502** increases rapidly to provide a decelerating force as the arm approaches the stop **454,** thereby damping the motion of the arm to prevent bouncing of the arm when engaging the stop. The arm **442** comes to rest at the stop **454** and the currents **500** and **502** reduce to a small holding current in each of the coils **458** and **460** to hold the arm at the stop **454.** A second time period **508** during which the arm **442** held at the stop **454** provides sufficient time to complete capture of the first image.

Similarly, a subsequent rising edge of the SYNCH signal **506** defines a start time of a third time period **510,** in which the current **502** causes an attractive force and the current **500** a repulsion force on the arm **442** to cause the arm to move toward the stop **456.** A time period **512** during which the arm **442** is at rest at the stop **456** defines a fourth time period **512,** which provides sufficient time to complete capture of the second image.

The modulator controller **480** also includes an output **486** for producing a synchronization signal (SYNC) for synchronizing operation of the image sensor **423,** if required. Alternatively, the output **486** may be configured as an input for receiving a synchronization signal generated by a controller associated with controlling the image sensor.

In another embodiment related to the embodiment shown in Figure **7****,** a compact illuminator (such as a light emitting diode (LED)) may be carried on the spatial modulator **410** so as to direct the illumination flux to illuminate the first and second portions of the field of view **404.**

In other embodiments, the spatial modulator **410** may be replaced by a solid state modulator that is configured to couple an illumination flux of light through first and second portions of the imaging path to illuminate respective first and second portions of the field of view by selectively actuating a modulator disposed in the imaging path to alternate between transmitting the first image while reflecting at least a portion of the illumination flux through the second portion of the imaging path to illuminate the first portion of the field of view, and transmitting the second image while reflecting at least a portion of the illumination flux through the first portion of the imaging path to illuminate the second portion of the field of view. Such solid state modulator that relies on the wavelength of illumination flux being different to the imaging wavelengths may be fabricated using dichroic modulator elements and or filter components.

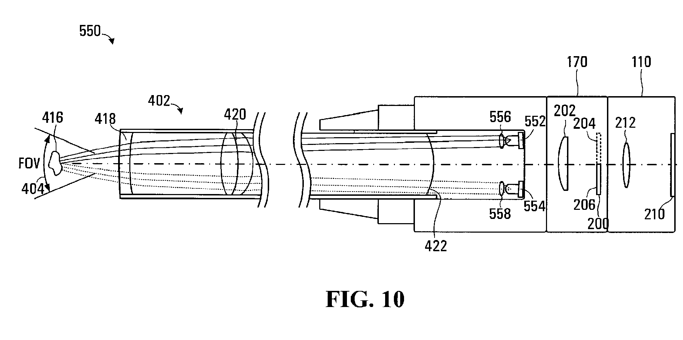

Referring to Figure **10****,** an apparatus for generating three dimensional image information according to a third embodiment of the invention is shown generally at **550.** The apparatus **550** has an optical system that is similarly configured to the optical system shown in Figure **7** and common reference numbers have been used to reference similar elements in the two embodiments. The optical system **402** has an imaging path disposed to capture light within the field of view **404** of the optical system, and the objective lens **418,** and a plurality of other lenses (of which a field lens **420** and a relay lens **422** are shown) form part of the imaging path. In this embodiment, the illumination coupler and spatial modulator shown in Figure **7** are replaced by a first illumination source **552** and a second illumination source **554.** The first illumination source **552** includes a lens **556** for gathering and directing light generated by the first illumination source and the second illumination source **554** includes a lens **558** for gathering and directing light generated by the second illumination source. In one embodiment each of the first and second illumination sources **552** and **554** may comprise a light emitting diode or a plurality of light emitting diodes. The apparatus **550** also includes an image modulator **170** and image sensor **110** that are similarly configured to the image modulator **170** and image sensor **110** shown in Figure **4****.**

In operation, the first and second illumination sources **552** and **554** are respectively configured to generate an illumination flux when activated in response to a control signal. The first and second illumination sources **552** and **554** thus alternate between illuminating a first portion of the field of view **404** and illuminating the second portion of the field of view. While the first portion of the field of view is illuminated, the image modulator **170** is actuated to transmit a first image through the imaging path. Similarly, while the second portion of the field of view is illuminated, the image modulator **170** is actuated to transmit a second image through the imaging path. As in the embodiments described above, the first and second images are together operable to represent three dimensional spatial attributes of objects within the field of view.

While specific embodiments of the invention have been described and illustrated, such embodiments should be considered illustrative of the invention only and not as limiting the invention as construed in accordance with the accompanying claims.

### EXAMPLES

1. A method for generating three dimensional image information through an optical system having an imaging path defined by a plurality of optical elements disposed to capture light within a field of view of the optical system, the method comprising: coupling an illumination flux through at least one illuminating portion of the imaging path to illuminate at least a portion of the field of view of the optical system; and receiving first and second images through respective first and second imaging portions of the imaging path, said first and second imaging portions being disposed adjacent to said at least one illuminating portion, said first imaging portion having a first perspective viewpoint within the field of view and said second imaging portion having a second perspective viewpoint within the field of view, said first and second images together being operable to represent three dimensional spatial attributes of objects within the illuminated field of view.
2. The method of example 1 wherein coupling said illumination flux through said at least one illuminating portion of the imaging path comprises coupling said illumination flux through at least one generally rectangular portion of the imaging path defined between two generally parallel lines extending through the imaging path.
3. The method of example 2 wherein coupling said illumination flux through said at least one generally rectangular portion of the imaging path comprises coupling said illumination flux through a generally rectangular portion surrounding an optical axis associated with the imaging path and wherein receiving said first and second images comprises receiving first and second images through respective first and second imaging portions of the imaging path disposed on either side of said generally rectangular portion.
4. The method of example 2 wherein coupling said illumination flux through said at least one generally rectangular portion of the imaging path comprises coupling said illumination flux through first and second generally rectangular portions disposed on either side of an optical axis associated with the imaging path and wherein receiving said first and second images comprises receiving first and second images through respective first and second imaging portions of the imaging path disposed between said first and second generally rectangular portions.
5. The method of example 1 wherein the plurality of optical elements have a generally circular cross-sectional shape and wherein coupling said illumination flux through said at least one illuminating portion of the imaging path comprises coupling said illumination flux through first and second segments of the imaging path disposed on either side of an optical axis associated with the imaging path and wherein receiving said first and second images comprises receiving first and second images through respective first and second imaging portions of the imaging path disposed between said first and second segments of the imaging path.
6. The method of example 1 wherein coupling said illumination flux through said at least one illuminating portion of the imaging path comprises coupling said illumination flux through at least one generally circular portion of the imaging path.
7. The method of example 6 wherein coupling said illumination flux through said at least one generally circular portion of the imaging path comprises coupling said illumination flux through a generally circular portion of the imaging path surrounding an optical axis of the imaging path and wherein receiving said first and second images comprises receiving first and second images through respective first and second imaging portions of the imaging path disposed on either side of said generally circular portion.
8. The method of example 1 wherein coupling said illumination flux through said at least one illuminating portion of the imaging path comprises coupling said illumination flux through at least one illuminating portion of a first optical element of the plurality of optical elements.
9. The method of example 8 wherein coupling said illumination flux through said at least one illuminating portion of said first optical element comprises coupling said illumination flux through at least one illuminating portion of at least one of: an objective lens; and a window disposed to transmit light from the field of view into the imaging path.
10. The method of example 9 wherein coupling said illumination flux through said at least one illuminating portion comprises coupling said illumination flux through a centrally disposed opening in said at least one of said objective lens and said window.
11. The method of example 1 wherein coupling said illumination flux through said at least one illuminating portion of the imaging path comprises receiving said illumination flux from an said illumination flux source and guiding said illumination flux to said at least one illuminating portion of the imaging path.
12. The method of example 11 wherein guiding said illumination flux comprises guiding said illumination flux through one of an optical fiber bundle and a waveguide.
13. The method of example 1 further comprising receiving said first and second images at an image sensor and causing said image sensor to generate first and second signals representing said respective first and second images.
14. The method of example 13 further comprising receiving said first and second signals at a display and causing said display to display said first and second images.
15. The method of example 14 further comprising selectively directing the first image to a left eye of an operator and selectively directing the second image to a right eye of the operator.
16. The method of example 15 wherein said display is operably configured to simultaneously display each of the first and second images and further comprising preventing the first image from reaching the right eye of the operator and preventing the second image from reaching the left eye of the operator while permitting the first image to reach the left eye of the operator and permitting the second image to reach the right eye of the operator.
17. The method of example 1 wherein receiving said first and second images through first and second imaging portions of the imaging path comprises alternating between preventing light from reaching said first imaging portion of the imaging path and preventing light from reaching said second imaging portion of the imaging path.
18. The method of example 1 wherein receiving said first and second images comprises simultaneously receiving said first and second images through said first and second imaging portions of the imaging path and further comprising alternating between: preventing light passing through the first imaging portion of the imaging path from being transmitted through the optical system; and preventing light passing through the second imaging portion the imaging path from being transmitted through the optical system.
19. The method of example 1 wherein receiving said first and second images comprises: simultaneously receiving said first and second images through said first and second imaging portions of the imaging path and simultaneously transmitting said first and second images through said optical system; simultaneously receiving said first and second images transmitted through said optical system at an image modulator; and causing said image modulator to selectively transmit said respective first and second images.
20. The method of example 19 wherein causing said image modulator to selectively transmit said respective first and second images comprises causing said image modulator to alternately: block light associated with said first image while transmitting light associated with said second image; and block light associated with said second image while transmitting light associated with said first image.
21. The method of example 19 wherein causing said image modulator to selectively transmit said respective first and second images comprises causing said image modulator to: modify a state of light associated with said first image to have a first optical state; modify a state of light associated with said second image to have a second optical state; and wherein said first and second optical states are operable to facilitate separation of said first and second images into first and second images at an image sensor disposed to receive said first and second images.
22. The method of example 21 wherein causing said image modulator to modify said state of light associated with said first and second images to have respective first and second optical states comprises causing said image modulator to modify a polarization state of said respective first and second images.
23. An apparatus for generating three dimensional image information, the apparatus comprising: an optical system having an imaging path disposed to capture light within a field of view of the optical system, said imaging path comprising at least one illuminating portion operably configured to couple an illumination flux to illuminate at least a portion of the field of view of the optical system; and an image modulator operably configured to cause first and second images to be received through respective first and second imaging portions of the imaging path, said first and second imaging portions being disposed adjacent to said at least one illuminating portion, said first imaging portion having a first perspective viewpoint within the field of view and said second imaging portion having a second perspective viewpoint within the field of view, said first and second images together being operable to represent three dimensional spatial attributes of objects within the illuminated field of view.
24. The apparatus of example 23 wherein said at least one illuminating portion comprises at least one generally rectangular portion of the imaging path defined between two generally parallel lines extending through the imaging path.
25. The apparatus of example 24 wherein said at least one generally rectangular portion of the imaging path comprises a generally rectangular portion surrounding an optical axis associated with the imaging path and said image modulator is operably configured to cause said first and second images to be received through respective first and second imaging portions of the imaging path disposed on either side of said generally rectangular portion.
26. The apparatus of example 24 wherein said at least one generally rectangular portion comprises first and second generally rectangular portions disposed on either side of an optical axis associated with the imaging path and wherein said image modulator is operably configured to cause said first and second images to be received through respective first and second imaging portions of the imaging path disposed between said first and second generally rectangular portions.
27. The apparatus of example 23 wherein the plurality of optical elements have a generally circular cross-sectional shape and wherein said at least one illuminating portion of the imaging path comprises first and second segments of the imaging path disposed on either side of an optical axis associated with the imaging path and wherein said image modulator is operably configured to cause said first and second images to be received through respective first and second imaging portions of the imaging path disposed between said first and second segments of the imaging path.
28. The apparatus of example 23 wherein said at least one illuminating portion of the imaging path comprises at least one generally circular portion of the imaging path.
29. The apparatus of example 28 wherein said at least one generally circular portion of the imaging path comprises a generally circular portion of the imaging path surrounding an optical axis of the imaging path.
30. The apparatus of example 23 wherein said at least one illuminating portion of the imaging path comprises at least one illuminating portion of a first optical element of the plurality of optical elements.
31. The apparatus of example 30 wherein said at least one illuminating portion of said first optical element comprises at least one illuminating portion of at least one of: an objective lens; and a window disposed to transmit light into the imaging path.
32. The apparatus of example 31 wherein said at least one illuminating portion of said objective lens comprises a centrally disposed opening in said at least one of said objective lens; and said window.
33. The apparatus of example 23 further comprising: an illumination source; and an illumination path operably configured to receive said illumination flux from said illumination source and to guide said illumination flux to said illuminating portion of the imaging path.
34. The apparatus of example 33 wherein said illumination path comprises one of an optical fiber bundle and a waveguide.
35. The apparatus of example 23 further comprising an image sensor for receiving said first and second images and generating first and second signals representing said respective first and second images.
36. The apparatus of example 35 further comprising a display for receiving said first and second signals and displaying said first and second images.
37. The apparatus of example 36 further comprising an optical processor operably configured to selectively direct the first image to a left eye of an operator and to selectively direct the second image to a right eye of the operator.
38. The apparatus of example 37 wherein said display is operably configured to simultaneously display each of the first and second images and wherein said optical processor is operably configured to prevent the first image from reaching the right eye of the operator and to prevent the second image from reaching the left eye of the operator while permitting the first image to reach the left eye of the operator and permitting the second image to reach the right eye of the operator.
39. The apparatus of example 23 wherein said image modulator is disposed to alternate between preventing light from reaching said first imaging portion of the imaging path and preventing light from reaching said second imaging portion of the imaging path.
40. The apparatus of example 23 wherein said optical system is operably configured to simultaneously receive said first and second images through said first and second imaging portions of the imaging path and wherein said image modulator is operably configured to alternate between: preventing light passing through the first imaging portion of the imaging path from being transmitted through the optical system; and preventing light passing through the second imaging portion the imaging path from being transmitted through the optical system.
41. The apparatus of example 23 wherein said optical system is operably configured to simultaneously receive said first and second images through said first and second imaging portions of the imaging path and to simultaneously transmit said first and second images through said optical system and wherein said image modulator is disposed to simultaneously receive said first and second images transmitted through said optical system and to selectively transmit said respective first and second images.
42. The apparatus of example 41 wherein said image modulator is operably configured to selectively transmit said respective first and second images by alternately : blocking light associated with said first image while transmitting light associated with said second image; and blocking light associated with said second image while transmitting light associated with said first image.
43. The apparatus of example 41 wherein said image modulator is operably configured to selectively transmit said respective first and second images by: modifying a state of light associated with said first image to have a first optical state; modifying a state of light associated with said second image to have a second optical state; and wherein said first and second optical states are operable to facilitate separation of said first and second images into first and second images at an image sensor disposed to receive said first and second images.
44. The apparatus of example 43 wherein said image modulator is operabiy configured to modify said state of light associated with said first and second images by modifying a polarization state of said respective first and second images.
45. A method for generating three dimensional image information using an optical system having an imaging path disposed to capture light within a field of view of the optical system, the method comprising alternating between: receiving a first image through a first portion of the imaging path while directing an illumination flux through a second portion of the imaging path to illuminate a first portion of the field of view of the optical system, said first portion of the imaging path having a first perspective viewpoint within the field of view; and receiving a second image through a second portion of the imaging path while directing said illumination flux through the first portion of the imaging path to illuminate a second portion of the field of view of the optical system, said second portion of the imaging path having a second perspective viewpoint within the field of view, said first and second images together being operable to represent three dimensional spatial attributes of objects within the field of view.
46. The method of example 45 wherein receiving said first and second images comprises receiving first and second images through an elongated imaging path operable to capture light from an inaccessible location.
47. The method of example 46 wherein said inaccessible location is a location within a living body.
48. The method of example 45 further comprising generating said illumination flux and wherein directing said illumination flux through said first and second portions of the imaging path to illuminate said respective first and second portions of the field of view comprises selectively actuating a modulator disposed in the imaging path to alternate between: transmitting said first image while reflecting at least a portion of said illumination flux through said second portion of said imaging path to illuminate said first portion of the field of view; and transmitting said second image while reflecting at least a portion of said illumination flux through said first portion of said imaging path to illuminate said second portion of the field of view.
49. The method of example 48 wherein selectively actuating said modulator comprises selectively actuating a mechanical shutter to move between: a first position in which the shutter blocks transmission of said second image through the imaging path while reflecting said illumination flux through said imaging path to illuminate the second portion of the field of view; and a second position in which the shutter blocks transmission of said first image through the imaging path while reflecting said illumination flux through said imaging path to illuminate the first portion of the field of view.
50. The method of example 49 wherein selectively actuating said modulator comprises selectively actuating a light valve to alternately: block transmission of said second image through the imaging path while reflecting said illumination flux through said imaging path to illuminate the second portion of the field of view; and block transmission of said first image through the imaging path while reflecting said illumination flux through said imaging path to illuminate the first portion of the field of view.
51. The method of example 45 wherein directing said illumination flux through said first and second portions of the imaging path to illuminate said respective first and second portions of the field of view comprises alternating between: selectively actuating a first illuminator disposed to illuminate said first portion of the field of view while selectively actuating a modulator to transmit said first image through said imaging path; and selectively actuating a second illuminator disposed to illuminate said second portion of the field of view while selectively actuating said modulator to transmit said second image through said imaging path.
52. The method of example 51 wherein selectively actuating said first and second illuminators comprises selectively actuating first and second illuminators disposed proximate an aperture plane of the optical system.
53. The method of example 51 wherein selectively actuating said first and second illuminators comprises selectively actuating respective first and second light emitting diodes.
54. The method of example 51 wherein selectively actuating said modulator comprises causing a shutter to move between first and second positions within said imaging path.
55. The method of example 45 wherein said first portion of the field of view of the optical system and the second portion of the field of view of the optical system have an overlapping region within the field of view.
56. The method of example 45 wherein directing said illumination flux through said respective first and second portions of the imaging path comprises receiving said illumination flux from an illumination source and selectively guiding said illumination flux to said respective first and second portions of the imaging path.
57. The method of example 56 wherein selectively guiding said illumination flux comprises guiding said illumination flux into an optical fiber bundle having first and second respective pluralities of fibers, said first plurality of fibers being disposed to illuminate said first portion of the field of view and said second plurality of fibers being disposed to illuminate said second portion of the field of view.
58. The method of example 45 further comprising selectively directing the first image to a left eye of the operator and selectively directing the second image to a right eye of the operator.
59. The method of example 58 wherein selectively directing comprises: presenting each of the first and second images to each of the left and right eyes of the operator; and preventing the first image from reaching the right eye of the operator and preventing the second image from reaching the left eye of the operator while permitting the first image to reach the left eye of the operator and the second image to reach the right eye of the operator.
60. The method of example 58 wherein selectively directing comprises alternating between permitting the first image to be transmitted through the optical system and permitting the second image to be transmitted through the optical system while alternating between selectively permitting the first image to reach the left eye of the operator and selectively permitting the second image to reach the right eye of the operator.
61. An apparatus for generating three dimensional image information, the apparatus comprising: an optical system having an imaging path disposed to capture light within a field of view of the optical system; an image modulator operably configured to cause first and second images to be received through respective first and second imaging portions of the imaging path, said first portion of the imaging path having a first perspective viewpoint within the field of view, said second portion of the imaging path having a second perspective viewpoint within the field of view; an illuminator operably configured to alternate between: directing said illumination flux through said second portion of the imaging path to illuminate a first portion of the field of view of the optical system while said first image is being received; and directing said illumination flux through said first portion of the imaging path to illuminate a second portion of the field of view of the optical system, while said second image is being received; said first and second images together being operable to represent three dimensional spatial attributes of objects within the field of view.
62. The apparatus of example 61 wherein the imaging path comprises an elongated imaging path operable to capture light from an inaccessible location.
63. The apparatus of example 62 wherein said inaccessible location is a location within a living body.
64. The apparatus of example 61 wherein said illuminator comprises an illumination source operable to generate an illumination flux and wherein said modulator is operably configured to alternate between: transmitting said first image while reflecting at least a portion of said illumination flux through said second portion of said imaging path to illuminate said first portion of the field of view; and transmitting said second image while reflecting at least a portion of said illumination flux through said first portion of said imaging path to illuminate said second portion of the field of view.
65. The system of example 64 wherein said modulator comprises a mechanical shutter operably configured to move between: a first position in which the shutter blocks transmission of said second image through the imaging path while reflecting said illumination flux through said imaging path to illuminate the second portion of the field of view; and a second position in which the shutter blocks transmission of said first image through the imaging path while reflecting said illumination flux through said imaging path to illuminate the first portion of the field of view.
66. The apparatus of example 65 wherein said modulator comprises a light valve operably configured to alternately: block transmission of said second image through the imaging path while reflecting said illumination flux through said imaging path to illuminate the second portion of the field of view; and block transmission of said first image through the imaging path while reflecting said illumination flux through said imaging path to illuminate the first portion of the field of view.
67. The apparatus of example 61 wherein said illuminator comprises: a first illuminator disposed to illuminate said first portion of the field of view while receiving said first image through said imaging path; and a second illuminator disposed to illuminate said second portion of the field of view while receiving said second image through said imaging path.
68. The apparatus of example 67 wherein said first and second illuminators are disposed proximate an aperture plane of the optical system.
69. The apparatus of example 67 wherein said first and second illuminators comprise respective first and second light emitting diodes.
70. The apparatus of example 67 wherein said modulator comprises a shutter operably configured to move between first and second positions within said imaging path.
71. The apparatus of example 61 wherein said first portion of the field of view of the optical system and the second portion of the field of view of the optical system have an overlapping region within the field of view.
72. The apparatus of example 61 wherein said illuminator comprises: an illumination source for generating said illumination flux; and a guide operable to selectively guide said illumination flux to said respective first and second portions of the imaging path.
73. The apparatus of example 72 wherein said guide comprises an optical fiber bundle having first and second respective pluralities of fibers, said first plurality of fibers being disposed to illuminate said first portion of the field of view and said second plurality of fibers being disposed to illuminate said second portion of the field of view.
74. The apparatus of example 61 further comprising means for selectively directing the first image to a left eye of the operator and means for selectively directing the second image to a right eye of the operator.
75. The apparatus of example 74 wherein said means for selectively directing comprises: means for presenting each of the first and second images to each of the left and right eyes of the operator; and means for preventing the first image from reaching the right eye of the operator and preventing the second image from reaching the left eye of the operator while permitting the first image to reach the left eye of the operator and the second image to reach the right eye of the operator.
76. The apparatus of example 74 wherein said modulator is operably configured to alternate between permitting the first image to be transmitted through the optical system and permitting the second image to be transmitted through the optical system and wherein said means for selectively directing comprises means for alternating between selectively permitting the first image to reach the left eye of the operator and selectively permitting the second image to reach the right eye of the operator.

## Claims

1. A method for generating three dimensional image information using an optical system having an imaging path disposed to capture light within a field of view of the optical system, the method comprising alternating between:
receiving a first image through a first portion of the imaging path while directing an illumination flux through a second portion of the imaging path to illuminate a first portion of the field of view of the optical system, said first portion of the imaging path having a first perspective viewpoint within the field of view; and
receiving a second image through a second portion of the imaging path while directing said illumination flux through the first portion of the imaging path to illuminate a second portion of the field of view of the optical system, said second portion of the imaging path having a second perspective viewpoint within the field of view, said first and second images together being operable to represent three dimensional spatial attributes of objects within the field of view.

2. The method of claim 1, wherein receiving said first and second images comprises receiving first and second images through an elongated imaging path operable to capture light from an inaccessible location.

3. The method of claim 2, wherein said inaccessible location is a location within a living body.

4. The method of claim 1, further comprising generating said illumination flux and wherein directing said illumination flux through said first and second portions of the imaging path to illuminate said respective first and second portions of the field of view comprises selectively actuating a modulator disposed in the imaging path to alternate between:
transmitting said first image while reflecting at least a portion of said illumination flux through said second portion of said imaging path to illuminate said first portion of the field of view; and
transmitting said second image while reflecting at least a portion of said illumination flux through said first portion of said imaging path to illuminate said second portion of the field of view.

5. The method of claim 4, wherein selectively actuating said modulator comprises selectively actuating a mechanical shutter to move between:
a first position in which the shutter blocks transmission of said second image through the imaging path while reflecting said illumination flux through said imaging path to illuminate the second portion of the field of view; and
a second position in which the shutter blocks transmission of said first image through the imaging path while reflecting said illumination flux through said imaging path to illuminate the first portion of the field of view.

6. The method of claim 5, wherein selectively actuating said modulator comprises selectively actuating a light valve to alternately: block transmission of said second image through the imaging path while reflecting said illumination flux through said imaging path to illuminate the second portion of the field of view; and block transmission of said first image through the imaging path while reflecting said illumination flux through said imaging path to illuminate the first portion of the field of view.

7. The method of claim 1, wherein directing said illumination flux through said first and second portions of the imaging path to illuminate said respective first and second portions of the field of view comprises alternating between:
selectively actuating a first illuminator disposed to illuminate said first portion of the field of view while selectively actuating a modulator to transmit said first image through said imaging path; and
selectively actuating a second illuminator disposed to illuminate said second portion of the field of view while selectively actuating said modulator to transmit said second image through said imaging path.

8. The method of claim 7, wherein selectively actuating said first and second illuminators comprises selectively actuating first and second illuminators disposed proximate an aperture plane of the optical system.

9. The method of claim 7, wherein selectively actuating said first and second illuminators comprises selectively actuating respective first and second light emitting diodes.

10. The method of claim 7, wherein selectively actuating said modulator comprises causing a shutter to move between first and second positions within said imaging path.

11. The method of claim 1, wherein said first portion of the field of view of the optical system and the second portion of the field of view of the optical system have an overlapping region within the field of view.

12. The method of claim 1, wherein directing said illumination flux through said respective first and second portions of the imaging path comprises receiving said illumination flux from an illumination source and selectively guiding said illumination flux to said respective first and second portions of the imaging path.

13. The method of claim 12, wherein selectively guiding said illumination flux comprises guiding said illumination flux into an optical fiber bundle having first and second respective pluralities of fibers, said first plurality of fibers being disposed to illuminate said first portion of the field of view and said second plurality of fibers being disposed to illuminate said second portion of the field of view.

14. The method of claim 1, further comprising selectively directing the first image to a left eye of the operator and selectively directing the second image to a right eye of the operator.

15. The method of claim 14, wherein selectively directing comprises:
presenting each of the first and second images to each of the left and right eyes of the operator; and preventing the first image from reaching the right eye of the operator and preventing the second image from reaching the left eye of the operator while permitting the first image to reach the left eye of the operator and the second image to reach the right eye of the operator.

16. The method of claim 14, wherein selectively directing comprises alternating between permitting the first image to be transmitted through the optical system and permitting the second image to be transmitted through the optical system while alternating between selectively permitting the first image to reach the left eye of the operator and selectively permitting the second image to reach the right eye of the operator.

17. An apparatus for generating three dimensional image information, the apparatus comprising:
an optical system having an imaging path disposed to capture light within a field of view of the optical system;
an image modulator operably configured to cause first and second images to be received through respective first and second imaging portions of the imaging path, said first portion of the imaging path having a first perspective viewpoint within the field of view, said second portion of the imaging path having a second perspective viewpoint within the field of view;
an illuminator operably configured to alternate between:
directing said illumination flux through said second portion of the imaging path to illuminate a first portion of the field of view of the optical system while said first image is being received; and
directing said illumination flux through said first portion of the imaging path to illuminate a second portion of the field of view of the optical system, while said second image is being received,
said first and second images together being operable to represent three dimensional spatial attributes of objects within the field of view.
